# EUROPÄISCHE PATENTSCHRIFT

(11) **EP 1 206 216 B1**
(45) Veröffentlichungstag und Bekanntmachung des Hinweises auf die Patenterteilung: **23.04.2003**
(21) Anmeldenummer: 00954535.1
(22) Anmeldetag: 19.07.2000
(51) Int. Cl.: A61B 5/15, A61M 5/315

(54) **SPRITZE**
SYRINGE
SERINGUE

(30) Priorität: 02.08.1999 DE 19936294
(43) Veröffentlichungstag der Anmeldung: 22.05.2002
(73) Patentinhaber: Müller, Hans, 81547 München (DE)
(72) Erfinder: Müller, Hans, 81547 München (DE)
(74) Vertreter: Fleuchaus, Leo, Dipl.-Ing.
(86) Internationale Anmeldenummer: EP0006892
(87) Internationale Veröffentlichungsnummer: WO01008557

(56) Entgegenhaltungen:
- WO-A-99/20562
- US-A- 3 834 387
- US-A- 4 093 108

## Beschreibung

Die Erfindung betrifft eine Spritze, bestehend aus einem Zylinder zur Aufnahme von Körperflüssigkeiten, der sich am vorderen Ende zu einer Steckspitze verengt und am hinteren Ende einen nach aussen abstehenden Halteflansch hat sowie einem an einem Kolbengestänge mit Griffplatte befestigten Kolben, der mit einem mit einer Kolbendichtung versehenen Kolbenkopf und einem in vorgebbaren Auszugspositionen verrastbaren Kolbengestänge im Zylinder verschiebbar ist.

Diese Spritze dient insbesondere als Aufnahmegefäß zur extrakorporalen Behandlung von Körperflüssigkeiten, wie zum Beispiel für eine UV-Behandlung (UVB) oder eine hämatogene Oxydationstherapie (HOT).

Bei der hämatogenen Oxydationstherapie wird das dem Körper des Patienten entnommene Blut mit einfachem oder aktiviertem Sauerstoff, sog. Singulett-Sauerstoff, oder anderen therapeutischen Gasen behandelt. Im folgenden werden der Einfachheit halber solche therapeutisch eingesetzten Gase mit dem Überbegriff "Sauerstoff" bezeichnet.

In der DE-PS 43 30 189 ist eine Behandlung von Blut mit UV-Strahlung beschrieben, wobei eine herkömmliche handbetätigte Spritze als Aufnahmebehälter für das Blut Verwendung findet.

Anstelle der herkömmlichen handbetätigten Spritzen sieht die DE-OS 39 32 109 für die Blutentnahme eine Spritze mit Rastvorrichtungen am Kolbengestänge vor, das ein Reinfundieren des Blutes verhindern soll. Diese Rastvorrichtung erlaubt auch eine Festlegung des Kolbenauszugs in vorgegebene Auszugspositionen. Eine Rastvorrichtung zum selben Zweck ist auch in den EP-A-354824, FR-2536285 und DE-GM-7935103 vorgesehen.

Die DE-GM 7935103 weiße in diesem Zusammenhang auch auf die Erzeugung eines der Flüssigkeitseinsaugung dienenden Vakuums vor oder kurz nach dem Einstechen der Nadel in die Vene hin. Dazu sind entsprechende Rasten und Gegenrasten beschrieben.

Üblicherweise werden zur Arbeitserleichterung als Ersatz derartiger Spritzen für die besagten Therapieverfahren die Körperflüssigkeiten beim Entnahmevorgang in Vakuumflaschen durch den in der Flasche herrschenden Unterdruck angesaugt. In diesen Vakuumflaschen kann die entnommene Körperflüssigkeit dann auch mit Sauerstoff behandelt werden, indem die Flasche zur Begasung über ein Kupplungselement und einen Verbindungsschlauch an eine Sauerstoffquelle angeschlossen wird. Anschließend wird die in der Vakuumflasche behandelte Körperflüssigkeit dem Patienten mittels eines bekannten Transfusionsbestecks reinfundiert.

Ein gravierender Nachteil solcher Vakuumflaschen ist die variable und von aussen nicht überprüfbare Qualität des in der Flasche herrschenden Unterdrucks. Bei mangelndem Unterdruck wird während der Entnahme der Körperflüssigkeit eine zu geringe Menge angesaugt bzw. dem Patienten entnommen. Eine derartig unvollständige Probe muss dann samt Flasche verworfen und die Entnahme mit einer neuen Vakuumflasche wiederholt werden. Dies stellt neben dem materiellen Aufwand eine unzumutbare Belastung für den Patienten dar.

Ein weiterer Nachteil solcher Vakuumflaschen ist ihr volumenbedingter Platzbedarf sowie die daraus resultierenden hohen Lager-, Transport- und auch Entsorgungskosten. Hinzu kommt eine hohe Bruchgefahr im Umgang mit den aus Glas hergestellten Vakuumflaschen.

Ein alternatives Aufnahmegefäß für eine Vorrichtung zur extrakorporalen Behandlung von Blut ist aus der DE-GM 94 21 606 bekannt. Hierbei wird zur Behandlung mit therapeutischen Gasen das Blut nicht in Vakuumflaschen, sondern in flexiblen Kunststoffbeuteln behandelt. Solche Kunststoffbeutel sind äußerst platzsparend. Da sie jedoch nicht vakuumisierbar sind, erfolgt die Blutentnahme lediglich schwerkraftbedingt. Dies führt zu einem erhöhten Zeitaufwand bei der Blutentnahme und bringt damit zusätzliche Unbequemlichkeiten für den Patienten mit sich.

Es ist daher Aufgabe der Erfindung, ein Aufhahmegefäß für Körperflüssigkeiten bereitzustellen, das die Nachteile der im Stand der Technik beschriebenen Blutbeutel vermeidet und eine Vakuumflasche *sowie eine mit Ansaugvakuum arbeitende Spritze* ersetzen kann. Aus Kostengründen sowie um eine möglichst universelle Verwendbarkeit zu gewährleisten, wird eine Lösung gesucht, die einen möglichst geringen apparativen Aufwand erfordert und vielseitig einsetzbar ist.

Eine solche die Aufgabe lösende Vorrichtung ist ausgehend von der eingangs erwähnten Spritze erfindungsgemäß dadurch gekennzeichnet, daß sich das Kolbengestänge kreuzförmig im wesentlichen über den ganzen Querschnitt sowie die ganze Innenlänge des Zylinders erstreckt und eine rohrartige Längsbohrung aufweist, welche im Kolbenkopf hinter der Kolbendichtung mit einem Rückschlagventil, vorzugsweise einem Entenschnabelventil, versehen ist, daß in der Kappendichtung zum Spritzeninnern Löcher ausgebildet sind, und daß die Längsbohrung an der Steckspitze am hinteren Ende sowie die Steckspitze der Spritze mit einem aufsetzbaren Ventil oder einem Lockstopfen verschließbar sind.

Eine vorteilhafte Ausführungsform der Erfindung realisiert besagtes Verrasten des Kolbengestänges dadurch, dass in der Aussenkante des kreuzförmigen Kolbengestänges ein oder mehrere Ausschnitte angebracht sind, und dass auf dem Halteflansch eine Arretierscheibe derart aufsteckbar bzw. in das Kolbengestänge derart einsteckbar ist, dass sich ein Eingriff in Ausschnitte des Kolbengestänges ergibt, wenn diese Ausschnitte sich in der Ebene der Arretierscheibe bzw. des Steckstegs befinden.

Eine weitere Ausführungsform der Erfindung sieht an den kreuzförmigen Kolbengestänge Spreizstege vor, welche beim Aufziehen der Spritze an der inneren Mantelfläche entlanggleiten und sich nach aussen abspreizen, sobald sie über dem Halteflansch übergreifen können.

Erfindungsgemäß sind die Einschnitte oder Spreizstege so am Kolbengestänge positioniert, dass sich das Volumen im Zylinder der Spritze im eingerasteten Zustand als definierte Größe bemisst. Vorzugsweise werden die Positionen der Ausschnitte so gewählt, dass sich Volumina von 10 ml bis 120 ml in bevorzugten Positionen einstellen lassen.

Schließlich sieht die Erfindung als weitere Ausgestaltung auch vor, daß an einem oder mehreren der kreuzförmig angeordneten Stege des Kolbengestänges kurze stegförmige und elastische Rastlaschen derart angebracht sind, daß sie parallel zu einem benachbarten Steg verlaufen und sich zumindest mit einem Teil der Außenkante bis zur Außenebene der kreuzförmigen Stege erstrecken, daß die Rastlaschen beim Einschieben des Kolbengestänges nach seitlichem Verbiegen in das Spritzeninnere verschiebbar sind, und daß die Rastlaschen beim Aufziehen der Spritze zur Aufrechterhaltung der Kolbenstellung über den Halteflansch zurückfedern.

Um die Spritze vakuumisieren zu können, ist die Steckspitze und das hintere Ende der Längsbohrung mit einem Ventil, vorzugsweise einem Einwegehahn, verschließbar. Wenn der Kolben herausgezogen wird, entsteht im Innern des Spritzenzylinders ein Unterdruck. Dieser Unterdruck im Spritzenzylinder wird aufrechterhalten, indem das Kolbengestänge erfindungsgemäß verrastet wird.

Diese verschiedenen Variationen der erfindungsgemäßen Spritze bieten einen sehr preiswerten und vorteilhaften Ersatz für Vakuumflaschen aus Glas. Dabei kann die Spritze für alle Einsatzarten von Vakuumflaschen verwendet werden. Da das Vakuum immer erst bei Bedarf durch das Aufziehen der Spritze geschaffen wird, entfallen auch die Schwierigkeiten, welche sich durch Vakuumverlust beim Lagern von Vakuumflaschen ergeben.

Durch die Verschließbarkeit der Steckspitze sowie das Vorsehen von Löchern in der Kappendichtung des Kolbenkopfs, die über eine Rückflußsperre mit der rohrartigen Längsbohrung kommunizieren, ist Spritze zur hämatogenen Oxydationstherapie bestens geeignet.

Diese Längsbohrung ist während der Blutabnahme mit einem Einweghahn oder einem Luerlockstopfen hinten verschließbar und hat hinter der Kappendichtung die Rückflußsperre, vorzugsweise ein Entenschnabelventil, um das Eindringen von Blut in die Längsbohrung zu vermeiden.

Die Vorteile und Merkmale der Erfindung ergeben sich auch aus der Beschreibung von Ausführungsbeispielen in Verbindung mit den Ansprüchen und der Zeichnung.
Es zeigen
- Fig. 1: einen Längsschnitt durch eine Spritze gemäß der Erfindung;
- Fig. 2: die Spritze gemäß Fig. 1 im aufgezogenen und verrasteten Zustand das Kolbengestänges;
- Fig. 3: einen Schnitt längs der Linie III-III der Fig. 2;
- Fig. 4: einen Längsschnitt durch eine weitere Ausführungsform der Erfindung;
- Fig. 5: eine Spritze gemäß Fig. 4 im aufgezogenen und verrasteten Zustand des Kolbengestänges;
- Fig. 6: einen Schnitt längs der Linie VI-VI der Fig. 5;
- Fig. 7: eine weitere Ausführungsform der Spritze mit am Kolbengestänge angebrachten Rastlaschen;
- Fig. 8: einen Schnitt längs der Linie IX-IX der Fig 7;
- Fig. 9: einen teilweisen Längsschnitt durch die Spritze gemäß Fig 8 mit verrastetem Kolbengestänge;
- Fig. 10: eine Verbindung der erfindungsgemäßen Spritze zur UV-Bestrahlung (UVB) bzw. hämatogenen Oxydationstherapie (HOT).

In der nachfolgenden Figurenbeschreibung sind gleiche Teile mit gleichen Bezugszeichen versehen.

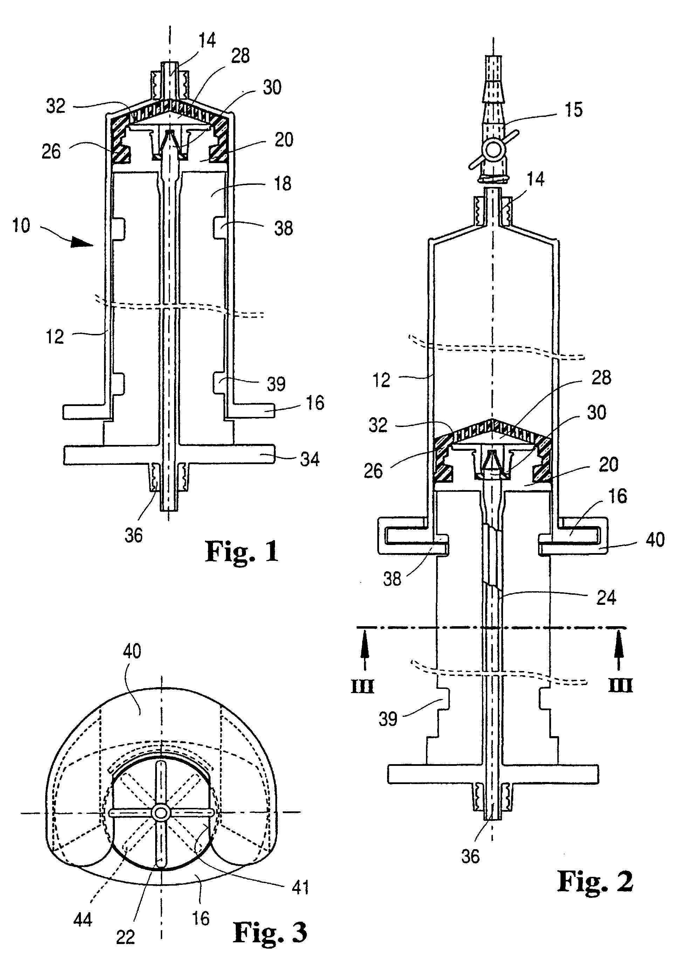

Die in den Fig. 1 bis 3 gezeigte Spritze 10 besteht aus einem Spritzenzylinder 12 zur Aufnahme von Körperflüssigkeit, der sich am vorderen Ende zu einer Steckspitze 14 verengt und am hinteren Ende einen nach aussen abstehenden Halteflansch 16 hat. Im Innern des Spritzenzylinders 12 ist ein mit einem Kolbengestänge 18 versehener Kolbenkopf 20 verschiebbar angebracht. Das Kolbengestänge besteht aus Kreuzstegen 22, in deren Zentrum eine rohrartige Längsbohrung 24 ausgebildet ist. Auf den Kolbenkopf 20 ist eine Kappendichrung 26 aufgesetzt, die an das sich verengende vordere Ende angepaßt ist. Zwischen der Kappendichtung 26 und dem Kolbenkopf befindet sich ein Freiraum 28, in welchen das vordere Ende der rohrartigen Längsbohrung mündet, auf deren vorderes Ende eine Rückflußsperre, vorzugsweise in Form eines Entenschnabelventils 30 aufgesetzt ist. Auch mit einem eingesetzten Bakterienfilter anstelle des Entenschnabelventils kann dieselbe Funktion erfüllt werden. In den verbleibenden Freiraum 28 münden kranzartig angeordnete Löcher 32, welche vorzugsweise vom äusseren Kranz nach innen verlaufend abnehmende Durchmesser haben. Am hinteren Ende der Kreuzstege 22 ist eine Griffplatte 34 ausgebildet, durch welche die Längsbohrung 24 verläuft und in einer Steckspitze 36 endet. Sowohl die vordere als auch die hintere Steckspitze 14 bzw. 36 sind vorzugsweise als Luer-Anschluß ausgebildet, in welchen ein Einweghahn 15, 56 einsetzbar ist.

Das kreuzstegförmige Kolbengestänge 18 ist an der Aussenkante mit Einschnitten 38, 39 versehen, die an vorgegebenen Positionen angebracht sind. Diese Positionen sind bestimmten Spritzenvolumina zugeordnet. Vorzugsweise sind die Einschnitte 38, 39 an Positionen vorgesehen, die beispielsweise ein Spritzenvolumen von 10 ml und 60 ml sowie bei großvolumigen Spritzen von 120 ml entsprechen.

Für eine eigens zur HOT-Behandlung vorgesehene Version der Spritze ist das aufziehbare Volumen im Spritzenzylinder 12 länger als das Kolbengestänge 18, um ein zusätzliches freies Volumen für das Aufschäumen zu erhalten.

Zur Verwendung der Spritze als Vakuumspritze werden auf die vordere und hintere Steckspitze 14 und 36 vorzugsweise jeweils ein Ventil in Form eines Einweghahns 15, 56 aufgesetzt. Ferner ist eine Arretierscheibe 40 vorgesehen, die einen U-förmigen Innenausschnitt 41 hat und, wie in Fig. 2 und 3 gezeigt, derart über den Halteflansch 16 schiebbar ist, daß sie in die Einschnitte 38 bzw. 39 eingreift und die aufgezogene Spritze in dieser zugeordneten Position festhält. Wenn beim Aufziehen der Spritze zum Beispiel der vorn und hinten aufgesetzte Einweghahn geschlossen ist, entsteht ein Unterdruck bzw. ein Vakuum im Spritzenzylinder, das durch die Verrastung der Position des Kolbengestänges mit der Arretierscheibe aufrechterhalten werden kann.

Zur Herstellung des Vakuums bei geschlossenem Spritzeneingang werden die Kreuzstege 22 des Kolbens in die in Fig. 3 gestrichelt dargestellte Position 44 gedreht und dann herausgezogen. Sobald die Einschnitte 38 bzw. 39 entsprechend dem gewünschten Vakuumvolumen in der Ebene der auf den Halteflansch 16 aufgeschobenen Arretierscheibe 40 liegen, wird der Kolben in die in Fig. 3 ausgezogen gezeigte Position der Kreuzstege 22 gedreht, womit sich die Einschnitte 38 bzw. 39 hinter der Arretierscheibe 40 verrasten und das Vakuum aufrechterhalten bleibt. Die auf diese Weise evakuierte Spritze kann jetzt wie eine bekannte Vakuumflasche für die herkömmliche Behandlung von Körperflüssigkeit benutzt werden, d.h. die beschriebene Spritze läßt sich als vollwertiger Ersatz für eine herkömmliche Vakuumflasche einsetzen.

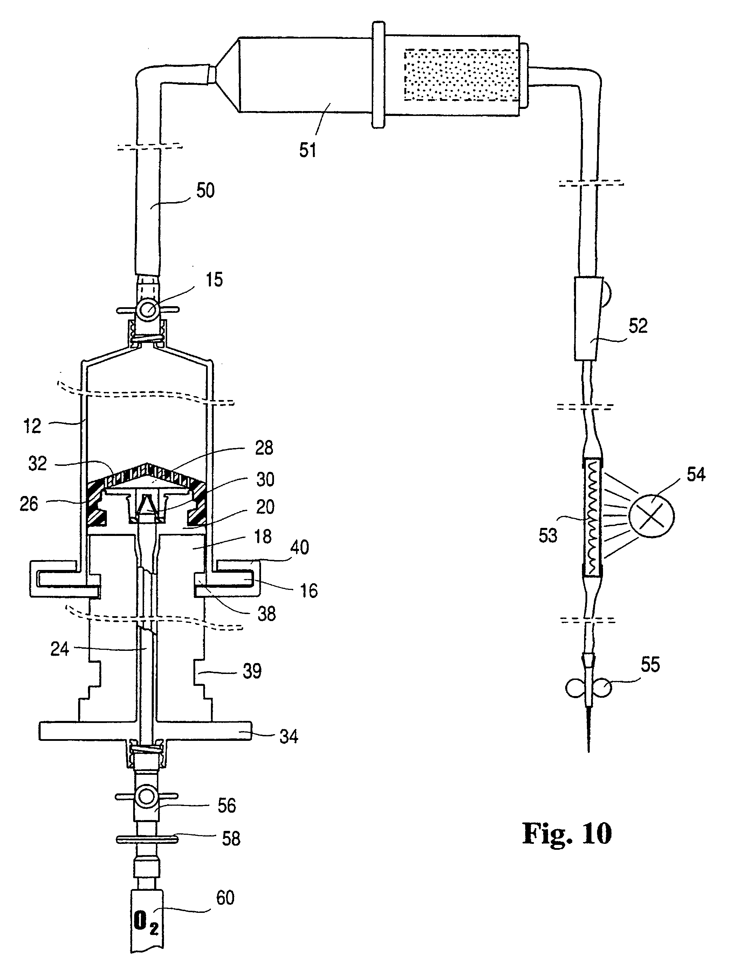

In Fig. 10 ist ein Transfusionsset für eine UVB- und/oder HOT-Behandlung dargestellt. Für eine derartige Behandlung wird einem Patienten zunächst Blut mit der erfindungsgemäßen Spritze entnommen. Dazu wird auf dem Luer-Anschluß an der Steckspitze 14 vorzugsweise der Einweghahn 15 und am hinteren Ende der Längsbohrung 24 der Einweghahn 56 befestigt. Bei geschlossenem Hahn wird durch manuelles Herausziehen des Kolbens aus dem Spritzenzylinder 12 ein Unterdruck aufgebaut. Der in der Position 44 der Kreuzstege 22 herausgezogene Kolben wird durch Drehen des Kolbengestänges 18 um ca. 45° in die ausgezogen dargestellte Position gebracht, so dass die Ausschnitte 39 bzw. 38 am Kolbengestänge in die auf den Halteflansch 16 aufsitzende Arretierscheibe 40 eingreifen. Durch dieses Einrasten wird ein Zurückgehen des Kolbens durch die Kraft des im Innern der Spritze befindlichen Vakuums vermieden. Die Maßnahme bewirkt, daß die Spritze, obwohl in ihrem Innern durch das mechanische Herausziehen des Kolbens ein Unterdruck erzeugt wurde, leicht und ohne Anstrengung handzuhaben ist.

Nachdem zunächst ein geringer Unterdruck durch das Eingreifen der Arretierscheibe 40 in die Einschnitte 39 aufgebaut worden ist, wird eine nicht dargestellte Kanüle auf den Einweghahn 15 aufgesetzt und bei geöffnetem Einweghahn aufgrund des in der Spritze bestehenden Unterdrucks ein Blutverflüssiger, wie zum Beispiel Natriumzitrat, in die Spritze aufgezogen.

Zur tatsächlichen Blutentnahme am Patienten wird bei erneut geschlossenem Einweghahn 15 die Kanüle vorzugsweise durch ein herkömmliches Transfusionsset aus Schlauch 50, Blutfilter 51, Rollklemme 52 und gegebenenfalls bei gleichzeitiger UVB-Behandlung Küvette 53 sowie eine Flügelkanüle 54 ersetzt. Nach einem weiteren Aufziehen des Kolbens und einer Verrastung des Kolbengestänges 18 in den Einschnitten 38 wird eine Vene des Patienten mit Hilfe der Flügelkanüle punktiert. Durch das Öffnen des Einweghahns 15 wird das Blut durch den in der Spritze herrschenden Unterdruck aktiv in die Spritze aspiriert. Wenn dem Patienten eine ausreichende Menge Blut entnommen ist, wird die Arretierung gelöst, um natriumzitrathaltiges Blut zurück in die Flügelkanüle zu drücken. Sobald der Einweghahn 15 danach geschlossen ist, wird zur HOT-Behandlung auf den Einweghahn 56 an der rückwärtigen Steckspitze 36 ein Bakterienfilter 58 aufgesteckt und dieses an eine Sauerstoffquelle 60 angeschlossen. Nach dem Öffnen des Einweghahns 56 kann Sauerstoff durch die Längsbohrung 24, das Entenschnabelventil 30 und die Löcher 32 in der Kappendichtung 26 in das Spritzeninnere einströmen. Das unter Druck einströmende Gas wird durch die Löcher 32 gepreßt und bewirkt ein Aufschäumen des Blutes. Anschließend wird bei geschlossenem Einweghahn 56 das Blut mit Sauerstoff verschüttelt, bis die Blutblasen zusammengefallen sind und die Sauerstoffbehandlung so oft wiederholt, bis die Blutfarbe zu hellrot umgeschlagen ist.

Die erfindungsgemäße Spritze mit dem HOT-behandelten Blut wird dann über das an den Einweghahn 15 angeschlossene Transfusionsset in herkömmlicher Weise reinfundiert. Dabei kann gegebenenfalls das begaste Blut über die Küvette 53 zur UV-Be-Strahlung in einer Bestrahlungsvorrichtung mit einem UV-Strahler 54 gerührt werden. Die Fließgeschwindigkeit des Blutes wird bei der Bestrahlung vorzugsweise durch die Rollklemme 52 eingestellt.

Nach abgeschlossener Behandlung des Blutes wird dieses über die Flügelkanüle 55 dem Patienten reinfundiert.

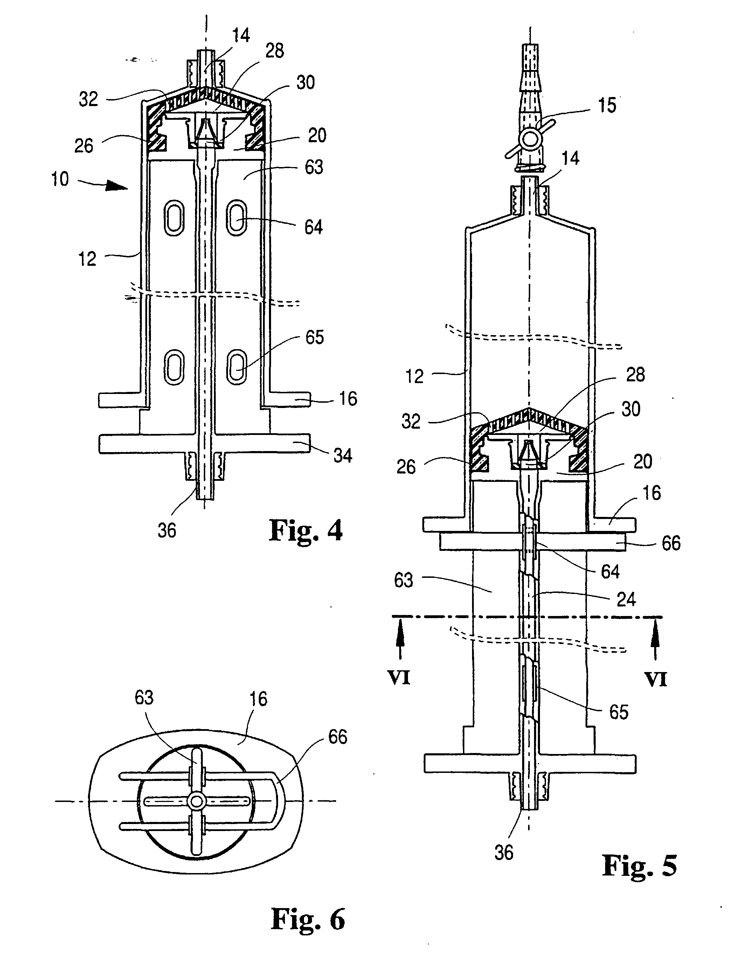

In den Fig. 4 bis 6 ist eine weitere Ausführungsform der Erfindung dargestellt: diese unterscheidet sich lediglich durch die Art der Arretierung des Kolbengestänges 63 am Halteflansch 16. Zur Fixierung des Kolbengestänges 63 sind in den Kreuzstegen 22 Ausschnitte bzw. Durchbrüche 64, 65 an den erwähnten vorgegebenen Positionen vorgesehen, durch welche ein U-förmiger Stecksteg 66 einsteckbar ist. um das Kolbengestänge 63 gegen den Halteflansch 16, wie in den Fig. 5 und 6 gezeigt, zu verrasten.

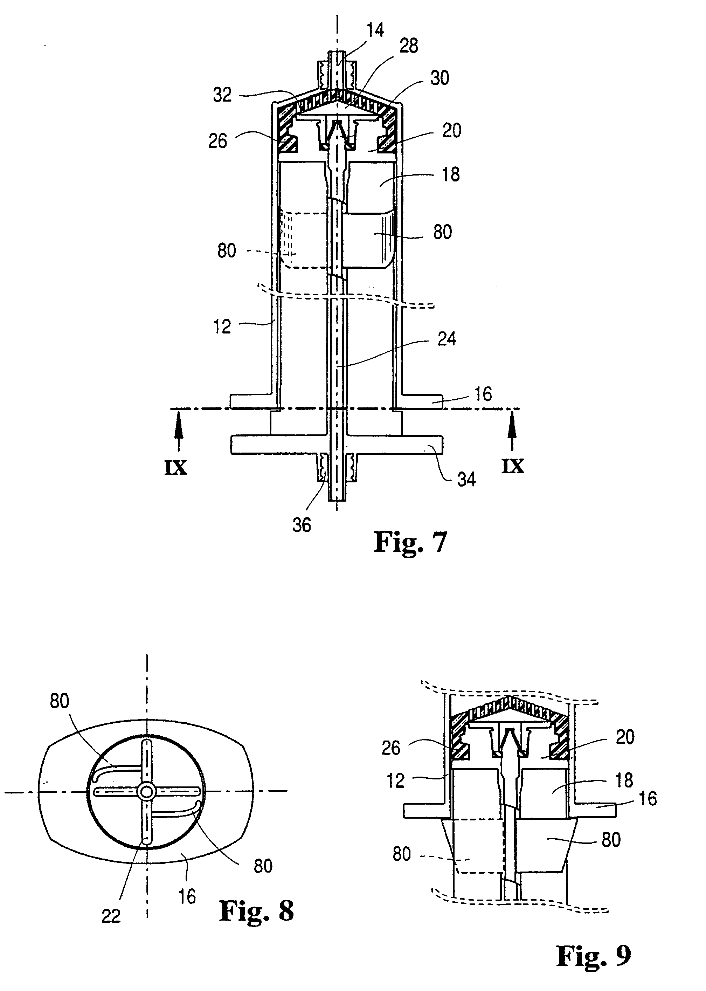

Schließlich ist in den Fig. 7 bis 9 eine besonders vorteilhafte Verrastung des Kolbengestänges 18 am Halteflansch 16 dargestellt. Zu diesem Zweck sind an vorzugsweise zwei Kreuzstegen 22 des Kolbengestänges verbiegbare Rastlaschen 80 angebracht, die in einem Abstand parallel zu einem Kreuzsteg verlaufen und zumindest gleich lang wie die Kreuzstege sind. Vorzugsweise sind sie zumindest mit der dem Kolbenkopf zugewandten Stirnseite etwas langer, so daß sie sich nach dem Aufziehen sicher am Halteflansch gemäß Fig. 9 verrasten.

Beim Einführen des Kolbengestänges in den Spritzzylinder lassen sich die Rastlaschen 80 leicht so weit verbiegen, daß sie, wie in Fig. 8 gezeigt, an der Innenfläche anliegen. Beim Herausziehen des Kolbengestänges federn die Rastlaschen zurück und gewährleisten eine sichere Verrastung.

Wie bei den zuvor beschriebenen Ausführungsformen können zusätzliche nicht dargestellte Rastlaschen am Kolbengestänge angebracht sein, um mehrere Rastpositionen vorzusehen.

## Patentansprüche

1. Spritze, bestehend aus einem Zylinder (12) zur Aufnahme von Körperflüssigkeiten, der sich am vorderen Ende zu einer Steckspitze (14) verengt und am hinteren Ende einen nach außen abstehenden Halteflansch (16) hat, sowie einem an einem sich kreuzförmig im wesentlichen über den ganzen Querschnitt sowie die ganze Innenlänge des Zylinders (12) erstreckenden Kolbengestänge (18, 63) mit Griffplatte (34) befestigten Kolben, der mit einem mit einer als Kappendichtung (26) ausgebildeten Kolbendichtung (26) versehenen Kolbenkopf (20) und einem in vorgebbaren Auszugspositionen verrastbaren Kolbengestänge im Zylinder verschiebbar ist,
**dadurch gekennzeichnet,**
**daß** das Kolbengestänge (18; 63) eine rohrartige Längsbohrung (24) aufweist, welche im Kolbenkopf (20) hinter der Kolbendichtung mit einer Rückflußsperre, vorzugsweise einem Entenschnabelventil (30), versehen ist,
**daß** in der Kappendichtung (26) zum Spritzeninnern Löcher (32) ausgebildet sind,
**daß** die Längsbohrung (24) am hinteren Ende des Kolbengestänges (18) in einer Steckspitze (36) endet,
und **daß** die Steckspitzen (14, 36) am vorderen und hinteren Ende der Spritze mit einem aufsetzbaren Ventil (15) oder einem Lockstopfen verschließbar sind.

2. Spritze nach Anspruch 1,
**dadurch gekennzeichnet,**
**daß** eine auf den Halteflansch (16) aufschiebbare Arretierscheibe (40) einen U-förmigen Innenausschnitt (41) hat, bei dem der Abstand der Schenkel kleiner als der Abstand einander gegenüberliegender Außenkanten des kreuzförmigen Kolbengestänges (18) ist,
und **daß** im Kolbengestänge angebrachte Einschnitte (38,39) durch Verdrehen des Kolbens über die Schenkel des U-förmigen Innenausschnitts (41) greifen.

3. Spritze nach Anspruch 1,
**dadurch gekennzeichnet,**
**daß** ein oder mehrere Ausschnitte (64, 65) in gegenüberliegenden Flächen des Kolbengestänges (63) angebracht sind,
**daß** im aufgezogenen Zustand der Spritze ein Stecksteg (66) in die Ausschnitte (64, 65) derart einsteckbar ist, daß sich der Stecksteg gegen den Halteflansch (16) abstützt und den Kolben im aufgezogenen Zustand festhält.

4. Spritze nach Anspruch 2 oder 3,
**dadurch gekennzeichnet,**
**daß** die Einschnitte (38, 39) bzw. die Ausschnitte (64, 65) an Positionen des kreuzförmigen Kolbengestänges angebracht sind, die ausgewählten Spritzenfüllungen von 1 ml bis 100 ml und/oder 1 ml bis 120 ml entsprechen.

5. Spritze nach Anspruch 1,
**dadurch gekennzeichnet,**
**daß** an einem oder mehreren der kreuzförmig angeordneten Stege des Kolbengestänges (18) kurze stegförmige und elastische Rastlaschen (80) derart angebracht sind, daß sie parallel zu einem benachbarten Steg verlaufen und sich zumindest mit einem Teil der Außenkante bis zur Außenebene der kreuzförmigen Stege erstrecken,
**daß** die Rastlachen (80) beim Einschieben des Kolbengestänges durch seitliches Verbiegen in das Spritzeninnere verschiebbar sind,
und **daß** die Rastlaschen (80) beim Aufziehen der Spritze zur Aufrechterhaltung der Kolbenstellung über den Halteflansch (16) zurückfedern.

6. Spritze nach einem oder mehreren der Ansprüche 1 bis 5 zur Verwendung als Vakuumbehälter,
**dadurch gekennzeichnet,**
**daß** zur Herstellung eines Vakuums im Spritzeninnern die Steckspitze (15) und das hintere Ende der Längsbohrung (24) verschlossen werden und die Spritze bis zum Verrasten aufgezogen wird.

7. Spritze nach einem oder mehreren der Ansprüche 1 bis 6,
**dadurch gekennzeichnet,**
**daß** zur Sauerstoffbehandlung des vom Vakuum in den Spritzenhohlraum aufgenommenen Blutes das hintere Ende der rohrartigen Längsbohrung (24) an eine Sauerstoffquelle (66) anschließbar ist, über welche Sauerstoff zum Aufschäumen des Blutes über die Rückflußsperre (30) und die Löcher (32) in der Kappendichtung (26) den Spritzenhohlraum einführbar ist.

## Claims

1. A syringe comprising a cylinder (12) for receiving bodily fluids, which tapers at the front end to a spigot (14) and has an outwardly projecting mounting flange (16) at its rear end, a piston, which is connected to a piston rod (18, 63), with an engagement plate (34), extending in a cruciform shape substantially over the entire cross-section and the entire internal length of the cylinder (12) and is movable in the cylinder with a piston head (20), provided with a piston seal (26) constructed in the form of a cap seal (26), and a piston rod, which is lockable in predeterminable withdrawn positions, **characterised in that** the piston rod (18; 63) has a tubular longitudinal bore (24), which is provided in the piston head (20) behind the piston seal with a non-return valve, preferably a duckbill valve (30), that holes (32) to the interior of the syringe are formed in the cap seal (26), that the longitudinal bore (24) terminates at the rear end of the piston rod (18) in a spigot (36) and that the spigots (14, 36) at the front and rear ends of the syringe may be sealed with a positionable valve (15) or a stop plug.

2. A syringe as claimed in Claim 1, **characterised in that** a locking disc (40), which may be pushed onto the mounting flange (16), has a U-shaped internal recess (41), in which the spacing of the limbs is smaller than the spacing of opposite outer edges of the cruciform piston rod (18) and that recesses (38, 39) provided in the piston rod engage over the limbs of the U-shaped internal recess (41) by rotating the piston.

3. A syringe as claimed in Claim 1, **characterised in that** one or more recesses (64, 65) are provided in opposing surfaces of the piston rod (63) and that in the extended state of the syringe a web (66) is insertable into the recesses (64, 65) such that the web bears against the mounting flange (16) and holds the piston in the extended state.

4. A syringe as claimed n Claim 2 or 3, **characterised in that** the recesses (38, 39) or the recesses (64, 65) are provided at positions on the cruciform piston rod which correspond to selected syringe fillings of 1 ml to 100 ml and/or 1 ml to 120 ml.

5. A syringe as claimed in Claim 1, **characterised in that** short, web-shaped and elastic locking lugs (80) are so mounted on one or more of the cruciform webs of the piston rod (18) that they extend parallel to an adjacent web and at least a portion of their outer edge extends to the outer plane of the cruciform webs, that the locking lugs (80) are movable into the interior of the syringe, when the piston rod is moved in, by virtue of lateral bending and that the locking lugs spring back over the mounting flange (16), when the syringe is extended, to maintain the piston position.

6. A syringe as claimed in one or more of Claims 1 to 5 for use as a vacuum container, **characterised in that** in order to produce a vacuum in the interior of the syringe, the spigot (15) and the rear end of the longitudinal bore (24) are sealed and the syringe is extended until locking occurs.

7. A syringe as claimed in one or more of Claims 1 to 6, **characterised in that**, for the purpose of oxygen treatment of the blood drawn into the syringe cavity by the vacuum, the rear end of the tubular longitudinal bore (24) is connectable to an oxygen source (26), via which oxygen may be introduced into the syringe cavity via the non-return valve (30) and the holes (32) in the cap seal (26) in order to foam the blood.

## Revendications

1. Seringue constituée par un cylindre (12) pour recueillir des liquides du corps, qui se réduit à son extrémité avant en un embout (14) et comporte dans la partie arrière une bride de retenue (16) débordant à l'extérieur, et par un piston fixé à une tige de piston (18, 63) muni d'un plateau de préhension (34) qui s'étend en forme de croix principalement sur toute la section transversale ainsi que sur toute la longueur intérieure du cylindre (12) et qui est déplaçable dans le cylindre avec une tête de piston (20) munie d'une garniture de piston (26) réalisée comme joint de coiffe (26) et une tige de piston pouvant s'encliqueter dans des positions de sortie prédéterminées,
**caractérisée**
**en ce que** la tige de piston (18, 63) présente un perçage longitudinal (24) de forme tubulaire qui est muni dans la tête de piston (20) derrière la garniture de piston d'un clapet antiretour, de préférence une soupape en bec de canard (30),
**en ce que** des orifices (32) débouchant à l'intérieur de la seringue sont réalisés dans le joint de coiffe (26),
**en ce que** le perçage longitudinal (24) dans la partie arrière de la tige de piston (18) se termine dans un embout (36),
et **en ce que** les embouts (14, 36) aux extrémités avant et arrière de la seringue peuvent être fermés au moyen d'une soupape démontable (15) ou d'un bouchon.

2. Seringue selon la revendication 1,
**caractérisée**
**en ce qu'**une rondelle d'arrêt (40) pouvant être déplacée sur la bride de retenue (16) présente un évidement interne en forme de U (41), dont l'écart des branches est plus petit que l'écart entre les bords externes opposés de la tige de piston en forme de croix (18),
et **en ce que** des crans (38,39) prévus sur la tige de piston passent pardessus les branches de l'évidement interne en forme de U (41) par rotation du piston.

3. Seringue selon la revendication 1,
**caractérisée**
**en ce qu'**une ou plusieurs encoches (64, 65) sont prévues dans des parois opposées de la tige de piston (63),
**en ce que** lorsque la seringue se trouve en l'état de sortie du piston, une barrette enfichable (66) peut être introduite dans les encoches (64, 65) de telle sorte que la barrette enfichable vient s'appuyer contre la bride de retenue (16) et maintient le piston en l'état de sortie.

4. Seringue selon la revendication 2 ou 3,
**caractérisée en ce que**
les crans (38,39) et les encoches (64,65) sont prévus à des positions de la tige de piston en forme de croix qui correspondent à des dosages de contenu de la seringue allant de 1 ml à 100 ml et/ou de 1 ml à 120 ml.

5. Seringue selon la revendication 1,
**caractérisée**
**en ce que** de courtes pattes d'arrêt (80) élastiques en forme de nervure sont prévues sur une ou plusieurs des nervures disposées en forme de croix de la tige de piston (18) de telle manière à s'étendre parallèlement à une des nervures contiguës et à rejoindre au moins par une partie du bord externe la surface externe des nervures en forme de croix,
**en ce que** lors de la rentrée de la tige de piston, les attaches (80) sont déplaçables dans l'intérieur de la seringue en se déformant latéralement,
**en ce que** lors de la sortie de la tige de piston, les attaches (80) se rabattent élastiquement au-delà de la bride de retenue (16) pour maintenir la position du piston.

6. Seringue selon une ou plusieurs des revendications 1 à 5 pour son utilisation comme récipient sous vide,
**caractérisée en ce que**
pour créer un vide dans l'intérieur de la seringue, l'embout (15) et la partie arrière du perçage longitudinal (24) sont fermés et le piston est extrait de la seringue jusqu'en position encliquetée.

7. Seringue selon une ou plusieurs des revendications 1 à 6,
**caractérisée en ce que**
pour traiter avec de l'oxygène le sang recueilli par succion dans le corps creux de la seringue, la partie arrière du perçage longitudinal de forme tubulaire (24) peut être reliée à une source d'oxygène (66) par laquelle peut être introduit de l'oxygène dans le corps creux de la seringue à travers le clapet antiretour (30) et les orifices (32) dans le joint de coiffe (26) afin de faire mousser le sang.
